# EUROPEAN PATENT SPECIFICATION

(11) **EP 3 917 416 B1**
(45) Date of publication and mention of the grant of the patent: **26.07.2023**
(21) Application number: 20704590.7
(22) Date of filing: 29.01.2020
(51) Int. Cl.: A61B 17/32

(54) **CONNECTOR FOR SURGICAL HANDPIECE**
VERBINDER FÜR EIN CHIRURGISCHES HANDSTÜCK
PIÈCE DE CONNEXION POUR PIÈCE À MAIN CHIRURGICALE

(30) Priority: 30.01.2019 US 201916262525
(43) Date of publication of application: 08.12.2021
(73) Proprietor: Integra LifeSciences Enterprises, LLLP, Princeton, NJ 08540 (US)
(72) Inventor: COTTER, Daniel J., Plainsboro, New Jersey 08536 (US); BERTORELLI, John, Plainsboro, New Jersey 08536 (US); KETELHOHN, Robert A., Hollis, New Hampshire 03049 (US); NIESKOSKI, Michael, Plainsboro, New Jersey 08536 (US); SHEEHAN, James, Plainsboro, New Jersey 08536 (US)
(74) Representative: Gill Jennings & Every LLP
(86) International application number: PCT/IB2020/050713
(87) International publication number: WO 2020/157682

(56) References cited:
- WO-A1-87/06116
- WO-A1-2018/051196
- WO-A2-94/15539
- WO-A2-98/25542
- WO-A2-2004/026150
- US-A- 5 222 937
- US-A- 6 117 151
- US-A1- 2013 331 872
- US-A1- 2014 135 804

## Description

### BACKGROUND OF THE INVENTION

The present invention relates to connectors for surgical handpieces, for example, handpieces in ultrasonic surgical aspirator systems for tissue ablation.

Ultrasonic aspiration has become the standard of care for removal of tumors and diseased tissue in neurosurgery and general surgery. Ultrasonic aspirators are used for ultrasonic fragmentation of tissue at an operation site and aspiration of the tissue particles and fluid away from the site. Typically, ultrasonic surgical aspirators include an ultrasonic transducer supported within a handpiece, an ultrasonically vibrating horn or tip operably connected to the ultrasonic transducer, and a sleeve or flue positioned about the horn. The horn includes a longitudinally extending central bore having one end located adjacent a distal tip and a second end located adjacent the proximal end of the horn. The proximal end of the horn is adapted to engage a vacuum source to facilitate aspiration of fluid. The flue is positioned about the horn to define an annular passage. Irrigation fluid is supplied through the annular passage around the horn to the surgical site where it mixes with blood and tissue particles and is aspirated through the bore in the horn. By mixing the irrigation fluid with the blood and tissue particles, coagulation of the blood is slowed down and aspiration thereof is aided. When the longitudinally vibrating tip in such an aspirator is brought into contact with tissue, it gently, selectively, and precisely fragments and removes the tissue. U.S. Patent Nos. 5,015,227 and 4,988,334 disclose such ultrasonic surgical devices. A known ultrasonic aspirator on the market is the CUSA^{®} Excel Ultrasonic Surgical Aspirator (Integra LifeSciences Corporation, Plainsboro, New Jersey, U.S.A.).

Examples of existing handpieces include CUSA Excel 23 kHz and 36 kHz Handpieces (Integra LifeSciences Corporation, Plainsboro, New Jersey, U.S.A.) and those described in U.S. Patent Nos. 4,223,676, 4,425,115 and 6,214,017. Those existing handpieces require user installed O-rings to provide sealing, support, and clasping functionality for the nosecone. The O-rings need to be installed by the user with each surgical procedure. The O-rings are small and difficult to install in operating room by practitioners wearing gloves, and the O-rings can be placed incorrectly or on the wrong mating geometry. In addition, the O-rings are different in diameter and could be selected and installed incorrectly. The O-rings for different handpieces could be selected incorrectly in manufacturing and assembly. In other existing devices, O-ring seals may be reusable and sterilizable but require periodic maintenance, and thus raise some concerns on ability to clean and sterilize over life.

WO 2018/051196 A1 discloses a surgical handpiece nosecone having an end overmold portion and/or an internal overmold portion. The end overmold portion is located at an end of the nosecone and compressed between the surgical handpiece housing and nosecone. The internal overmold portion is positioned radially about the nosecone on the inner surface to provide a fluid tight seal that prevents ingress of irrigation fluid into the housing.

US 2014/0135804 A1 discloses ultrasonic and electro surgical devices. The disclosed embodiments include ultrasonic blades comprising various grasping features, devices configured to prevent ingress of surgical matter, e.g., fluid and tissue, in the space between an ultrasonic blade and an inner or outer tube distal of the distal seal, alternate closure mechanisms for ultrasonic devices, ultrasonic transducer rotation limiters to limit the rotation of an ultrasonic transducer, ultrasonic transducer rotation systems to provide unlimited continuous rotation of an ultrasonic device, integrated ultrasonic instmment electrically connected to provide RF spot coagulation with an ultrasonic (RF) generator, and coated ultrasonic/RF blades.

US 2013/331872 A1 discloses a system, delivery device and method delivers ultrasonic energy to a target musculoskeletal tissue site. In some embodiments, the delivery device includes a stainless steel needle joined to a horn. In some embodiments, the stainless steel needle is joined to the horn using a heating process or a brazing process.

WO 87/06116 A1 discloses an ultrasonic surgical aspirator having an improved irrigation/aspiration manifold and components to facilitate quick connections of the irrigation and aspiration lines to a handpiece. In combination with a handle assembly having an ultrasonic vibrator for vibrating an ultrasonic tip and a nosecone, a nosecone extension defining a rotating joint for connection to the nosecone in an articulating joint for connection to a rigid or flexible flue. The nosecone extension further defines an irrigation cowl having an irrigation port for receiving an irrigation connector for connecting the irrigation fluid to the nosecone extension for passage of irrigation fluid through the nosecone extension and a flue about the surgical tip. An aspiration tubulation connector adapted to securely fit a slot through the nosecone and nosecone extension connects an aspiration port on the tip to the aspiration tube for connection to a pump into a discard.

US 5222937 A discloses an ultrasonic treatment apparatus includes an ultrasonic vibration generating portion for generating ultrasonic vibrations, an amplifying portion for amplifying the ultrasonic vibrations generated by the ultrasonic vibration generating portion, a vibration transmitting member for transmitting the ultrasonic vibrations amplified by the amplifying portion, and a connecting member for connecting the vibration transmitting member to the amplifying portion such that the central axes of the vibration transmitting member and of the amplifying portion intersect at a predetermined angle. The generated ultrasonic vibration includes nodes and loop portions between adjacent nodes, and both a connecting portion between the connecting member and the amplifying portion and a connection portion between the connecting member and the vibration transmitting member are formed in the vicinity of a position at which a loop portion of the ultrasonic vibration lies.

Hence, those skilled in the art have recognized a need for a surgical handpiece connector with improved ease of use. Embodiments of the present invention fulfill this need and others.

### SUMMARY OF THE INVENTION

The invention is defined by independent claims 1 (apparatus) and 11 (method). Further embodiments of the invention are defined in the dependent claims. Embodiments which are not covered by the claims are only used for illustrative purposes and do not form part of the invention. No surgical methods form part of the invention. According to the invention there are provided apparatuses, systems and methods as set out in the appended claims. Embodiments of the present invention provide a connector for surgical handpiece, such as a nosecone, that incorporates an overmolding technology. More specifically, the nosecone eliminates user installed O-rings and enhances ease of use. The J-lock clasp of the nosecone and its seal is incorporated to a single proximal overmolded soft polymer and the distal diametrical seal is accomplished with a separate overmolded ring seal.

In the invention, a connection apparatus for attachment of members of a medical device comprises a first member having a first body and a first connecting section. In various embodiments, the first connecting section may have a first end face and an inner surface. In some embodiments, a second member may have a second body, a second connecting section and a shoulder at the junction between the second body and the second connecting section. Moreover, in some embodiments, the second connecting section may have a second end face and an outer surface shaped for telescoping into the first connecting section. In various embodiments, the outer surface may have a groove extending from the second end face towards the shoulder. In some embodiments, the first member may have a nub on the inner surface extending radially inward for engaging the groove. In various embodiments, the first member may have an end overmold portion at least partially covering the first end face and positioned to bear against the shoulder, whereby the end overmold portion may be compressed between the first body and the second body.

In addition, in various embodiments, the first member may have more than one nub on the inner surface arranged radially about the first connecting section, and the second member may have more than one groove on the outer surface arranged radially about the second connecting section, and each nub may be positioned to engage with each groove. In some embodiments, the end overmold portion may be made of a thermoplastic elastomer. In various embodiments, the first member may be a nosecone and the second member may be a surgical handpiece housing. Moreover, in some embodiments, the groove may be J-shaped. In various embodiments, the nub may be sphere-shaped. In some embodiments, the connection apparatus may comprise an internal overmold portion positioned radially about the inner surface of the first member.

In various embodiments, a method for attaching members of a medical device may comprise the steps of providing a first member having a first body and a first connecting section. In some embodiments, the first connecting section may have a first end face, an inner surface, a nub on the inner surface extending radially inward, and an end overmold portion at least partially covering the first end face. In various embodiments, the method may include providing a second member having a second body, a second connecting section and a shoulder at the junction between the second body and the second connecting section, the second connecting section may have a second end face and an outer surface, the outer surface may have a groove extending from the second end face towards the shoulder. In some embodiments, the method may include telescoping the second connecting section into the first connecting section. In various embodiments, the method may include locating the groove in the outer surface of the second connecting section. Moreover, in some embodiments, the method may include positioning the nub to engage the groove and moving the nub in the groove towards the shoulder until the end overmold portion contacts the shoulder whereby the end overmold portion may be compressed between the first body and the second body.

In some embodiments, the method may include providing more than one nub on the inner surface radially about the first connecting section. In various embodiments, the method may include providing more than one groove on the outer surface arranged radially about the second connecting section, wherein each nub may be positioned to engage with each groove. In some embodiments, the method may include positioning each nub to engage each groove. Moreover, in some embodiments, the method may include moving each nub in each groove towards the shoulder until the end overmold portion contacts the shoulder. In addition, in some embodiments, the overmold portion may be made of a thermoplastic elastomer. In some embodiments, the first member may be a nosecone and the second member may be a surgical handpiece housing. In various embodiments, the groove may be J-shaped. In some embodiments, the nub may be sphere-shaped. Moreover, in some embodiments, an internal overmold portion may be positioned radially about the inner surface of the first member.

In various embodiments, an ultrasonic aspirator system for fragmenting tissue and removing fragmented tissue may include a surgical handpiece comprising a housing, a nosecone attached to the housing, and a transducer mounted within the housing. The surgical tip is be connected to the transducer. In various embodiments, an irrigation system is connected to the handpiece for supplying irrigation fluid adjacent the surgical site for suspending fragmented tissue. An aspirating system is connected to the handpiece for aspirating fluid and tissue fragmented at the surgical site. The nosecone has an inner surface and an outer surface, and has an internal overmold portion on the inner surface may be positioned radially about the nosecone to provide a fluid tight seal that prevents ingress of irrigation fluid into the housing.

In addition, in various embodiments, the nosecone may further comprise an end overmold portion in contact with the housing to provide a seal between the housing and the nosecone. In some embodiments, the nosecone may comprise at least one nub on an inner surface extending radially inward. In various embodiments, the housing may be configured to attach to the nosecone and may have a groove on an outer surface extending proximally from a distal end of the housing. Further, in some embodiments, the nosecone may further comprise an end overmold portion at least partially covering a proximal end of the nosecone and positioned to bear against the housing. In various embodiments, the internal overmold portion may be made of a thermoplastic elastomer. In some embodiments, the nosecone may have a plurality of recessed lobes on the outer surface, the lobes being circumferentially spaced about a longitudinal axis. In various embodiments, the nosecone may have three recessed lobes spaced symmetrically on the outer surface to be grasped by a user.

In accordance with the invention, there is provided a connection apparatus for attachment of members of a medical device. The connection apparatus comprises a first member having a first body and a first connecting section, the first connecting section having a first end face and an inner surface; a second member having a second body, a second connecting section and a shoulder at the junction between the second body and the second connecting section, the second connecting section having a second end face and an outer surface shaped for telescoping into the inner surface of the first connecting section, the outer surface having a groove extending from the second end face towards the shoulder; wherein the first member has a nub on the inner surface extending radially inward for engaging the groove; and wherein the first member has an end overmold portion at least partially covering the first end face and positioned to bear against the shoulder, whereby the end overmold portion is compressed between the first body and the second body.

In more detailed aspects, in the connection apparatus, the first member has more than one nub on the inner surface arranged radially about the first connecting section, and the second member has more than one groove on the outer surface arranged radially about the second connecting section, and each nub is positioned to engage with each groove.

In accordance with the present invention, a method for attaching members of a medical device is provided. The method comprises providing a first member having a first body and a first connecting section, the first connecting section having a first end face, an inner surface, a nub on the inner surface extending radially inward, and an end overmold portion at least partially covering the first end face; providing a second member having a second body, a second connecting section and a shoulder at the junction between the second body and the second connecting section, the second connecting section having a second end face and an outer surface, the outer surface having a groove extending from the second end face towards the shoulder; telescoping the second connecting section into the first connecting section; locating the groove in the outer surface of the second connecting section; positioning the nub to engage the groove; and moving the nub in the groove towards the shoulder until the end overmold portion contacts the shoulder whereby the end overmold portion is compressed between the first body and the second body.

In accordance further aspects of embodiments of the present invention, there is provided an ultrasonic aspirator apparatus for fragmenting tissue and removing fragmented tissue. The ultrasonic aspirator apparatus comprises a surgical handpiece comprising a housing, a nosecone attached to the housing, and a transducer mounted within the housing; a surgical tip connected to the transducer; an irrigation system connected to the handpiece for supplying irrigation fluid adjacent the surgical site for suspending fragmented tissue; and an aspirating system connected to the handpiece for aspirating fluid and tissue fragmented at the surgical site; wherein the nosecone has an inner surface and an outer surface, and has an internal overmold portion on the inner surface positioned radially about the nosecone to provide a fluid tight seal that prevents ingress of irrigation fluid into the housing. The ultrasonic aspirator apparatus may additionally comprise an end overmold portion in contact with the housing to provide a seal between the housing and the nosecone.

The nosecone may have a plurality of recessed lobes on the outer surface, the lobes being circumferentially spaced about a longitudinal axis. For example, the nosecone may have a tri-lobe configuration on the outer surface to be grasped by a user.

In addition, in some embodiments, a connection apparatus for attachment of members of a medical device may include a nosecone having opposing first and second ends. In various embodiments, the connection apparatus may include a flue having opposing first and second ends. In some embodiments, the flue may have a base on the first end and an overmold portion on the second end. In various embodiments, the overmold portion of the flue may have a projection extending radially inward from an inner surface thereof. Moreover, in some embodiments, the second end of the nosecone releasably engages the first end of the flue.

In some embodiments, the nosecone may include a first and second overmold portion at each first end and second end, respectively. In various embodiments, the second overmold portion of the nosecone extends both radially outward from an outer surface to engage a groove of the base of the flue and radially inward from an inner surface. In some embodiments, the second overmold portion of the nosecone at least partially covers an end face of the second end of the nosecone. In addition, in various embodiments, the overmold portion of the flue includes an irrigation port. In some embodiments, the overmold portion of the flue may be more translucent than the base of the flue. In various embodiments, the first end of the flue may extend along a first axis and the second end of the flue may extend along a second axis different from the first axis.

In addition, in various embodiments, an ultrasonic aspirator apparatus for fragmenting tissue and removing fragmented tissue may include a surgical handpiece comprising a housing, a nosecone attached to the housing, a flue attached to the nosecone, and/or a transducer mounted within the housing. In some embodiments, the apparatus may include a surgical tip connected to the transducer via an internal horn. In various embodiments, the apparatus may include an irrigation system connected to the handpiece for supplying irrigation fluid adjacent the surgical site for suspending fragmented tissue. Moreover, in some embodiments, the apparatus may include an aspirating system connected to the handpiece for aspirating fluid and tissue fragmented at the surgical site. In some embodiments, the flue may include a base and one or more overmold portions defining an inner surface and an outer surface. In some embodiments, at least one of the overmold portions may project inwardly from the inner surface radially about the surgical tip to provide a fluid tight seal that prevents ingress of irrigation fluid into at least a portion of the base.

In some embodiments, the surgical tip may include a bone tip. Moreover, in various embodiments, the nosecone may include a base and one or more overmold portions. In some embodiments, the one or more overmold portions may be positioned on opposing ends of the base. In various embodiments, the one or more overmold portions of the nosecone radially inwardly engages the internal horn and/or radially outwardly engages the base of the flue. In addition, in some embodiments, the one or more overmold portions may cover an end face on each one of the opposing ends of the base of the nosecone. In various embodiments, the flue may include a groove within the inner surface of the base of the flue. In some embodiments, the one or more overmold portions of the nosecone may engage the groove of the flue. In various embodiments, the overmold portion of the flue may taper towards a free end of the flue away from the base of the flue.

In addition, in various embodiments, a method for attaching members of a medical device may include providing a nosecone having one or more overmold portions on opposing ends of a base. In some embodiments, the method may include providing a flue having one or more overmold portions on at least one end of a base, wherein at least one of the overmold portions project radially inwardly from an inner surface of the flue. In various embodiments, the method may include providing a handpiece having a body. In some embodiments, the method may include providing a tip. In various embodiments, the method may include connecting the nosecone to the handpiece. In addition, in some embodiments, the method may include connecting the flue to the nosecone. In various embodiments, the method may include sealingly engaging the overmold portion of the flue projecting radially inwardly with the tip to provide a fluid tight seal that prevents ingress of irrigation fluid towards the nosecone.

In some embodiments, the nosecone may be connected to the handpiece and the flue, the one or more overmold portions of the nosecone engages both the handpiece and the flue. In various embodiments, when the nosecone is connected to the handpiece and the flue, the one or more overmold portions of the nosecone engages an internal horn. In some embodiments, the method may include temporarily expanding the one or more overmold portions of the flue to pass over the tip when connecting the flue to the nosecone. In addition, in various embodiments, the method may include contracting the overmold portion to a rest position. In some embodiments, connecting the nosecone to the flue may include engaging the one or more protrusions of the nosecone with a circumferential groove within the inner surface of the flue.

Other features and advantages of the embodiments of the present invention will become more apparent from the following detailed description of the embodiments, when taken in conjunction with the accompanying exemplary drawings.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Embodiments of the present invention are described herein with reference to the drawings, in which:
FIG. 1 is a perspective view of an ultrasonic apparatus in accordance with embodiments of the present invention;
FIG. 2 is a perspective view of a handpiece with a nosecone in accordance with embodiments of the present invention;
FIG. 3 is a longitudinal-sectional view of a portion of the ultrasonic apparatus of FIG. 1;
FIG. 4 is a perspective view of an ultrasonic horn;
FIG. 5 is a perspective view of a nosecone fully assembled to a handpiece and supporting a flue (the flue tube is not shown in this drawing);
FIG. 6 is a perspective view of a nosecone in accordance with embodiments of the present invention;
FIG. 7 is a cross-sectional view taken along line A-A of FIG. 6.
FIG. 8 is a top plan view of the nosecone of FIG. 6;
FIG. 9 is a bottom plan view thereof;
FIG. 10 is a front elevational view thereof;
FIG. 11 is a distal end view thereof;
FIG. 12 is a proximal end view thereof;
FIG. 13 is a cross-sectional view taken along line B-B of FIG. 9;
FIG. 14 is a bottom plan view of the nosecone of FIG. 6 without the overmold portions;
FIG. 15 is a cross-sectional view taken along line E-E of FIG. 14
FIG. 16 is a cross-sectional view taken along line C-C of FIG. 15
FIG. 17 is a cross-sectional view taken along line D-D of FIG. 15
FIG. 18 shows the handpiece housing and nosecone in a dissembled state;
FIG. 19 is a perspective view of the handpiece housing;
FIG. 20 is a side view of the handpiece housing of FIG. 19;
FIG. 21 is an enlarged perspective view of the distal end of the handpiece housing of FIG. 19;
FIG. 22 is a cross-sectional view taken along line F-F of FIG. 20;
FIG. 23 is a cross-sectional view taken along line G-G of FIG. 20;
FIG. 24 is a partial longitudinal-sectional view of the handpiece housing and nosecone in an assembled state;
FIG. 25 is a perspective view of a portion of the handpiece housing and nosecone in an assembled state;
FIG. 26 is a perspective view of another embodiment of an ultrasonic apparatus in accordance with embodiments of the present invention;
FIG. 27 is a longitudinal-sectional view of the ultrasonic apparatus of FIG. 26;
FIG. 28 is a perspective view of a flue of the ultrasonic apparatus of FIG. 27;
FIG. 29 is a perspective view of the flue of the ultrasonic apparatus of FIG. 28 illustrating one or more overmold portions exploded from a base portion;
FIG. 30 is cross-sectional view taken along line 30-30 of FIG. 26;
FIG. 31 is perspective view of the base portion of the flue of FIG. 29;
FIG. 32 is perspective view of the overmold portion of the flue of FIG. 29;
FIG. 33 is perspective view of a nosecone of the ultrasonic apparatus of FIG. 28;
FIG. 34A is a perspective longitudinal-sectional view of the nosecone of the ultrasonic apparatus of FIG. 33 illustrating one or more overmold portions exploded from a base portion;
FIG. 34B is a perspective longitudinal-sectional view of the nosecone of the ultrasonic apparatus of FIG. 33;
FIG. 35 is an end view of the nosecone taken along line 35-35 of FIG. 34B;
FIG. 36 is a longitudinal-sectional view of the nosecone of the ultrasonic apparatus of FIG. 33;
FIG. 37 is cross-sectional view taken along line 37-37 of FIG. 36; and
FIG. 38 is an end view of the nosecone taken along line 38-38 of FIG. 36.

### DETAILED DESCRIPTION OF THE PREFERRED EMBODIMENTS

Embodiments of the presently disclosed connectors for surgical handpieces will now be described in detail with reference to the drawings, in which like reference numerals designate identical or corresponding elements in each of the several views. As used herein, the term "distal" refers to that portion of the instrument, or component thereof which is farther from the user while the term "proximal" refers to that portion of the instrument or component thereof which is closer to the user during normal use. The terms "ultrasonic horn," "ultrasonic tip," "ultrasonic surgical tip," "surgical tip", "horn" and "tip" are used herein interchangeably.

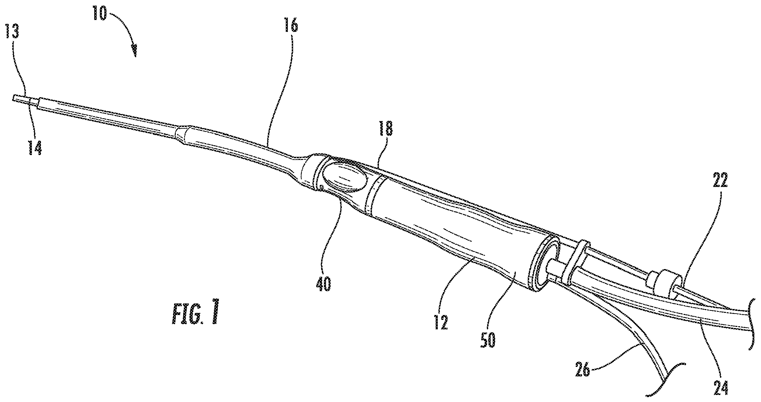

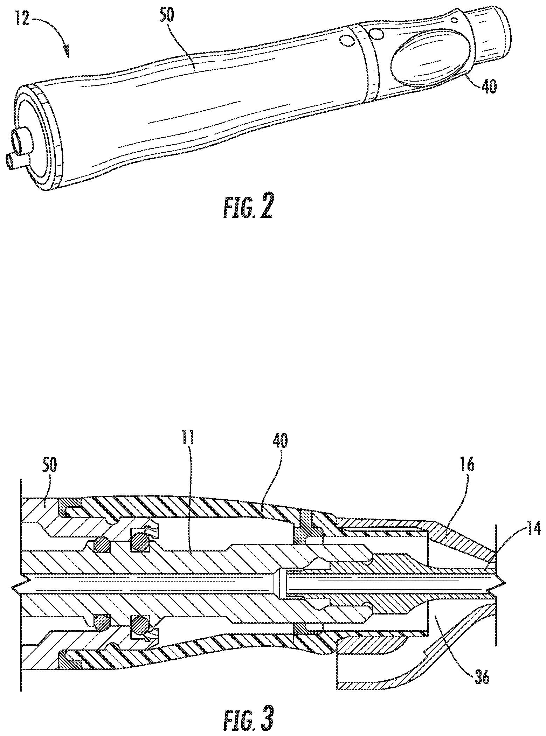

Referring now to FIGS. 1-3, one embodiment of the presently disclosed apparatus for ultrasonically fragmenting and aspirating tissue is shown. Generally an ultrasonic surgical apparatus 10 includes a handpiece 12 for use by a surgeon to direct fragmentation. The handpiece 12 encases a transducer (not shown) on which a surgical tip or ultrasonic horn 14 is fastened. The ultrasonic horn can be powered by the transducer and be ultrasonically actuated to fragment tissue and suction effluent via a central channel. A distal end portion 13 of the ultrasonic horn 14 extends beyond a distal end of a flue 16. Ultrasonic horn 14 is vibrated to fragment tissue during surgery. The ultrasonic horn may be made of titanium or other conventional materials known in the art.

A cooling and irrigation system which provides cooling fluid to the ultrasonic horn 14 is provided for maintaining temperature within an acceptable range. The handpiece 12 includes a housing 50, which may be formed of a sterilizable plastic, metal or other suitable materials or a combination thereof. The flue 16 provides a path for irrigation fluid or liquid and connects to the distal end of the handpiece 12. The flue 16 typically connects to the handpiece 12 via a nosecone 40. The flue 16 may include or attach to a flue tube 18. The nosecone 40 connects to the housing 50 and covers the internal ultrasonic horn 14.

An irrigation tube 22 connects to the flue tube 18 up-stream and supplies irrigation fluid through the flue tube 18 to an operative site during surgery. An aspiration tube 24 provides suction and a path for aspiration from the operative site to a collection canister (not shown). Alternatively, the aspiration tube may be mounted outside of the housing 50. An electrical cable 26 provides power to the apparatus or provides switching connections.

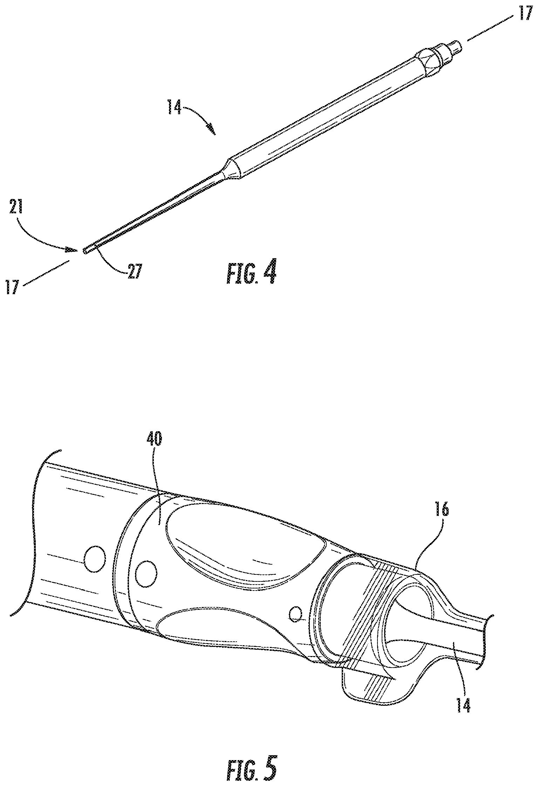

FIG. 4 illustrates an embodiment of an ultrasonic tip or ultrasonic horn 14, which is suitable for use with the above-described ultrasonic surgical apparatus for fragmenting and aspirating tissue. The ultrasonic horn has a throughbore 17 and a preaspiration hole or transverse bore 27. Although the ultrasonic horn as shown is a stepped horn, it is known that there are ultrasonic horns that are not stepped.

The ultrasonic horn 14 is substantially circular and disposed within the flue 16. During operation of the ultrasonic apparatus 10, irrigation fluid is supplied through the irrigation tube 22 and flue tube 18 into the flue 16. The flue 16 and the ultrasonic horn 14 define an annular cavity 36 therebetween. Irrigation fluid is supplied from flue 16 through the annular cavity 36 to the distal end of the ultrasonic horn 14. A transverse bore is formed in preaspiration holes 27 near the distal end of the ultrasonic horn 14 and communicates with the throughbore 17. The irrigation fluid is drawn from preaspiration holes 27 and the surgical site into an inlet 21 of the throughbore 17 along with fragmented tissue, blood, etc., and is removed from the surgical site via the throughbore 17 and the aspiration tube 24. The transverse bore provides an alternate route for fluid to enter throughbore 17 when inlet 21 becomes clogged. The nosecone 40 attaches to the housing 50 and covers internal portion of the ultrasonic horn 14.

In a more detailed aspect, irrigation liquid, for example saline, is necessary to cool the surgical tip and site of tissue fragmentation. This irrigation liquid may be provided to the flue with a peristaltic pump at a rate as low as 2 to 3 ml/min, which is only typically about a drip or two a second. The irrigation liquid is supplied at the proximal end of the ultrasonic horn. The irrigation liquid progresses to near the distal end of the ultrasonic horn, where two preaspiration holes, which may each have a 0.381 mm (0.015 inch) diameter for example, suction a majority, perhaps 90-95%, of the irrigation through the holes connecting the outside horn diameter to the central suction channel. This action of irrigation and suction supports a contiguous cooling circuit for the vibrating titanium metal and it also helps to wet effluent such as blood and tissue in the central channel. Some irrigation is also favorable to cooling the surgical site, improving coupling to tissue, and affording cavitation necessary to emulsification and aspiration of tissue, such as tumors.

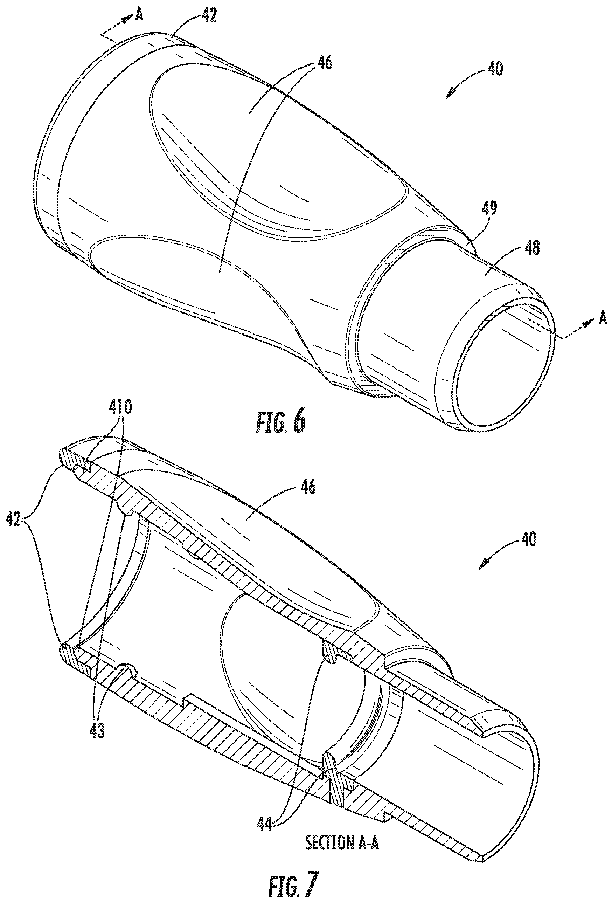

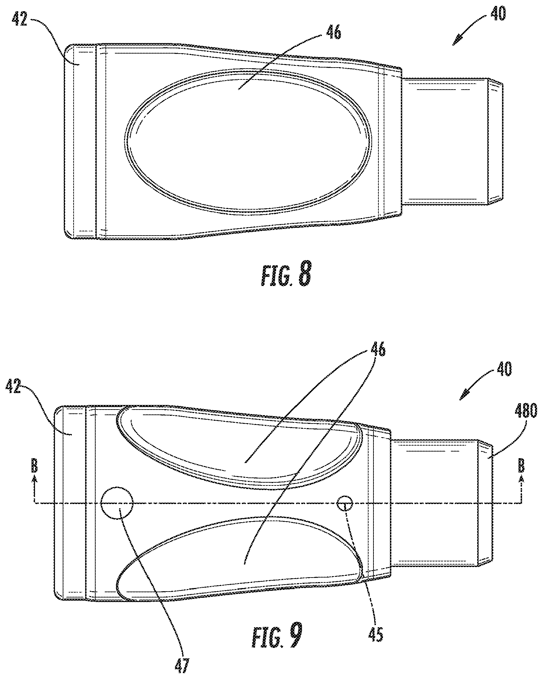

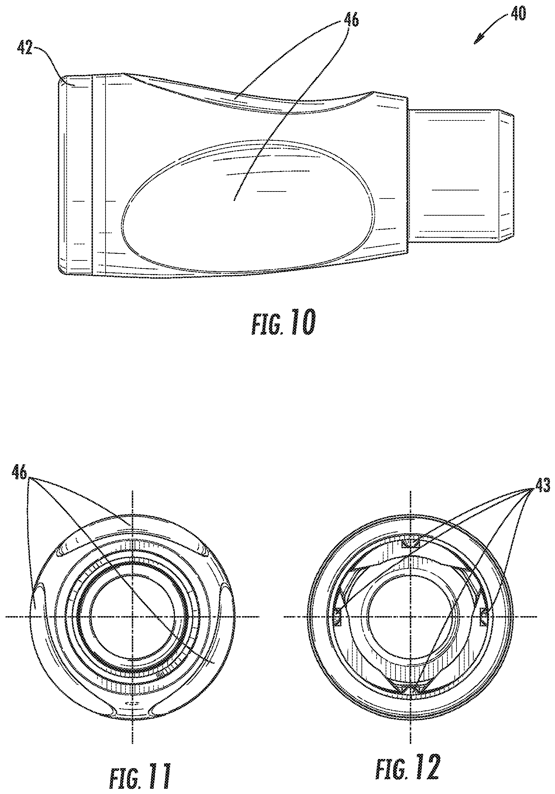

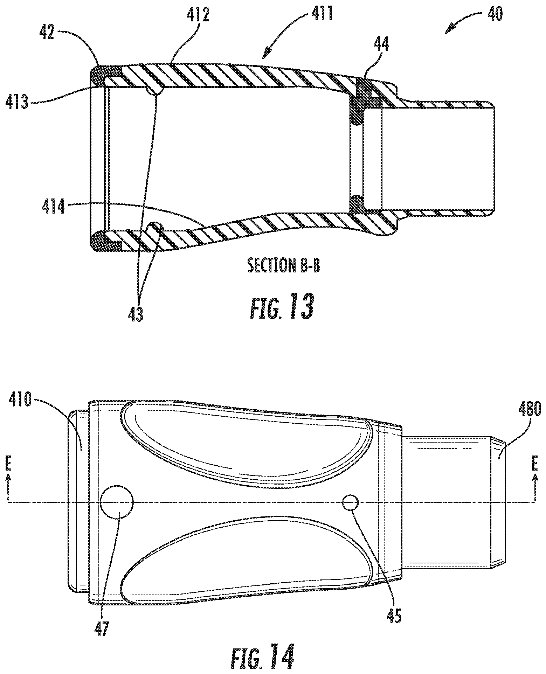

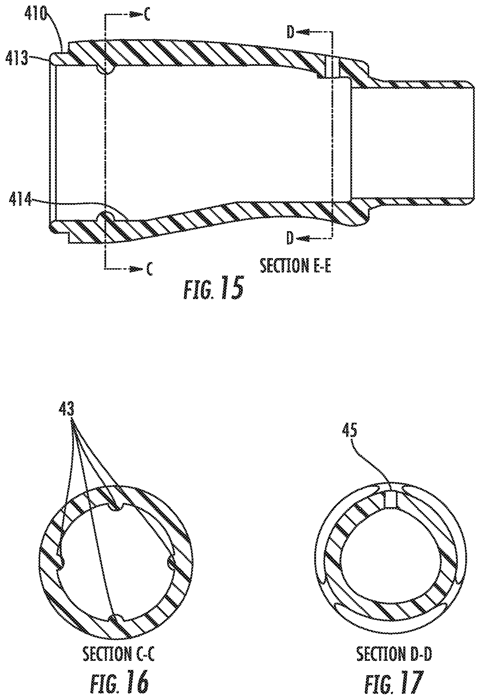

The nosecone 40 will be described in more detail with reference to FIGS. 6-17. The form of the nosecone is generally a cylindrical taper having a generally circular cross-section and a neck 48 at the narrower end of the taper. The tapered nosecone body transitions to the neck 48 through a nosecone shoulder 49. The neck 48 has a constant diameter that is smaller than the diameter of the narrower end of the taper. The neck 48 may have a beveled end 480. It provides a surface area for holding the flue 16. Alternatively, the nosecone may be substantially cylindrical and may have a cross-section that is generally oval or of another suitable shape.

The nosecone 40 may have a plurality of recessed lobes 46 on the outer surface. The lobes are circumferentially spaced, symmetrically or asymmetrically, about a longitudinal axis. The recessed lobes 46 may each have a recessed area of a shape suitable to be grasped by a user with fingers. For example, the recessed area may be oval or circular. In an exemplary embodiment, along the tapered section, three lobes, 120 degrees apart, provide finger grip placement. The tri-lobe configuration on the outer surface of the nosecone provides an ergonomic hand grip area that makes it easy for a user to grasp and hold the handpiece in hand and thus enhances comfort for the user.

An internal overmold portion 44, located proximal to the neck 48, serves as a primary fluid seal. The overmold portions eliminate the need for O-rings and thus enhance ease of assembly. The overmolding may be made from a medically comparable thermoplastic elastomer, for example polypropylene or other conventional materials used in gaskets, stoppers and seals as known in the art. A thermoplastic elastomer is a plastic polymeric material that becomes pliable or moldable above a specific temperature and solidifies upon cooling. The materials of the nosecone body and overmold portions are selected for adequate adherence and withstand of sterilization. Once fully assembled, the internal overmold portion 44 of the nosecone 40 conforms to the internal ultrasonic horn 14 to prevent fluid ingress into the handpiece 12.

The nosecone 40 has an end overmold portion 42 at the large diameter open end. The end overmold portion 42 is a part of the coupling mechanism that affixes the nosecone 40 to the housing 50. The coupling mechanism can be used not only for connecting the housing and nosecone components of surgical handpieces, but also for attachment of members of other medical devices or other apparatus.

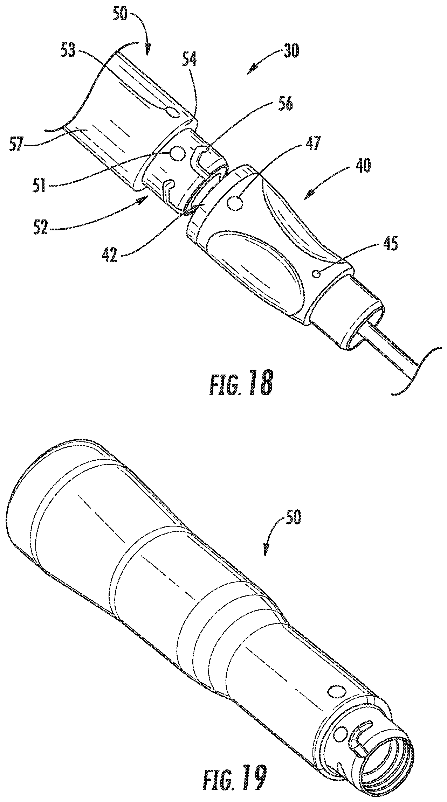

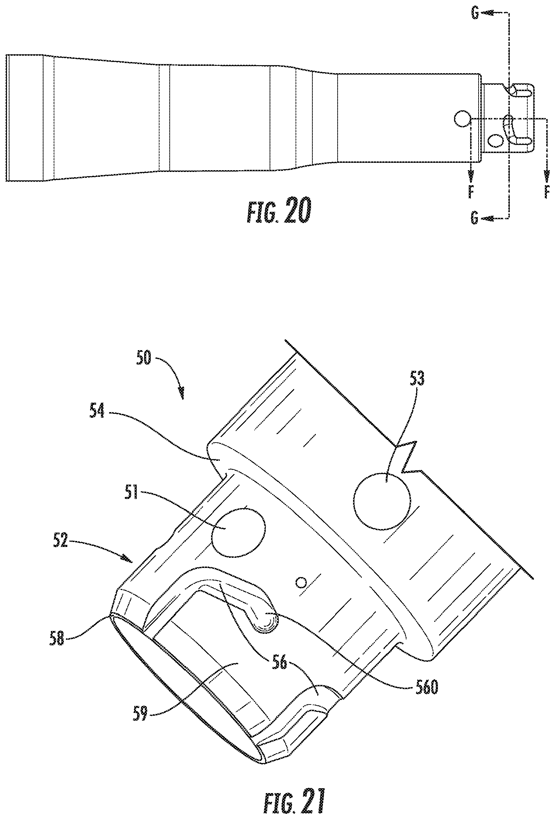

Turning now to FIGS. 18-23, in a general aspect, embodiments may provide a connection apparatus 30 for attachment of members of a medical device. The connection apparatus 30 comprises a first member, for example, a nosecone 40, and a second member, for example, a housing 50. The first member or nosecone 40 has a first body 411 and a first connecting section 412. The first member or nosecone may further comprise a lip 410 extending from the first connecting section (or nosecone connecting section) 412. The first connecting section has a first end face 413 and an inner surface 414. The second member or housing 50 has a second body 57, a second connecting section (or housing connecting section) 52 and a shoulder 54 at the junction between the second body 57 and the second connecting section 52. The second connecting section has a second end face 58 and an outer surface 59 shaped for telescoping into the inner surface of the first connecting section 412. The outer surface 59 comprises a groove 56 extending from the second end face 58 towards the shoulder 54. The groove 56 may be a curved or angled groove, such as a J-shaped groove, forming a moving track for the nub 43, with a rest position 560 at the end of the track, located between the shoulder 54 and the second end face 58. The first member or nosecone 40 comprises a nub 43 on the inner surface 414 extending radially inward for engaging the groove 56. The first member or nosecone 40 has an end overmold portion 42 at least partially covering the first end face and positioned to bear against the shoulder 54, whereby the end overmold portion 42 is compressed between the first body 411 and the second body 57.

The J-shaped groove may comprise a plurality of segments. For example, it may have three segments. The first segment extends from the second end face of the housing towards the shoulder of the housing and is generally perpendicular to the second end face. The second segment is at an angle relative to the first segment, for example, at an angle of about 15 to about 75 degrees, and preferably between about 30 to about 60 degrees. The third segment constitutes the rest position, which is shorter than the first segment and the second segment. It may be at an angle relative to the second segment. It is contemplated that the rest position may simply be the end of the second segment without being at any apparent angle relative to the second segment.

The end overmold portion 42 may be present on the outer diameter or inner diameter of the nosecone connecting section or both diameters. If a lip 410 exists on the nosecone, the end overmold portion may be present on the outer diameter of outer diameter or inner diameter of the lip 410 or both diameters. In the exemplary embodiment as shown in FIG. 7, the end overmold portion 42 is positioned at least partially on the outer diameter of the lip 410 and flush with the outer surface of the nosecone 40.

The first member 40 may have more than one nub on the inner surface 414, arranged radially about the first connecting section 412, and the second member 50 may have more than one groove 56 on the outer surface arranged radially about the second connecting section 52. The nubs 43 may be spaced symmetrically or asymmetrically apart, and the grooves are spaced accordingly such that each nub is positioned to engage with a respective groove. In the exemplary embodiment as shown, the first member 40 has four nubs 43 on its inner surface evenly spaced approximately 90 degrees apart, and the second member 50 has four grooves 56 on its outer surface evenly spaced approximately 90 degrees apart.

In the embodiment as shown, the first member is a nosecone and the second member is a surgical handpiece housing. It is contemplated that the first member may be a surgical handpiece housing and the second member may be a nosecone.

In operation, a user positions each nub 43 to engage a respective groove 56 and moves each nub 43 in the groove 56 towards the shoulder 54 of the housing 50 until the end overmold portion 42 contacts the shoulder 54. The grooves may be generally evenly spaced apart on the housing about a longitudinal axis. Likewise, the nubs may be generally evenly spaced apart on the inner surface of the nosecone about a longitudinal axis accordingly for engaging the grooves on the housing.

The inside of the nosecone is hollow to provide clearance space in order for it to slip over the ultrasonic horn 14 and connect to the housing 50. In the exemplary embodiment as shown in the drawings, four nubs 43, in the form of spheres that are about 1.524 mm (0.06 inches) in diameter, form part of the coupling mechanism that affixes the nosecone 40 to the housing 50. The spheres may have a diameter in the range of about 0.254 mm to 2.54 mm (about 0.01 to about 0.10 inches), for example, in the range of about 5.08 mm to 20.32 mm (about 0.2 to about 0.8 inches). The nubs may be in the form of other raised structures suitable for engaging with the grooves.

The overmold portions are preferably made of a material that has elastomeric properties of having a low Young's Modulus and flexibility. A proper material should also be able to achieve a strong, stable bond to the substrate. It also needs to be able to be injected into a mold in a semi-fluid or fluid state at an elevated temperature and to remain on the substrate and retain its strength and provides elasticity after it has cooled and solidified. In a preferred embodiment, the body of the nosecone is made from glass-filled polypropylene (PP) which has appropriate strength, stiffness, melting temperature and ability to adhere with overmold material (thermoplastic elastomer). In another preferred embodiment, the overmolding is made from a healthcare thermoplastic elastomer grade used in gaskets, stoppers and seals. It has the ability to bond to a variety of substrates, for example PP. Compression set, reseal capability, and an ability to be sterilized under autoclave and ethylene oxide are some of the factors to be considered in selecting materials for the design.

There are several identification markers on the apparatus for the coupling mechanism. The housing has a housing identification marker 53 on the housing body 57 and another connection identification marker 51 on the housing connecting section 52. The nosecone 40 has a nosecone identification marker 47. The identification markers may be in any form, shape and color as desired, such as white dots.

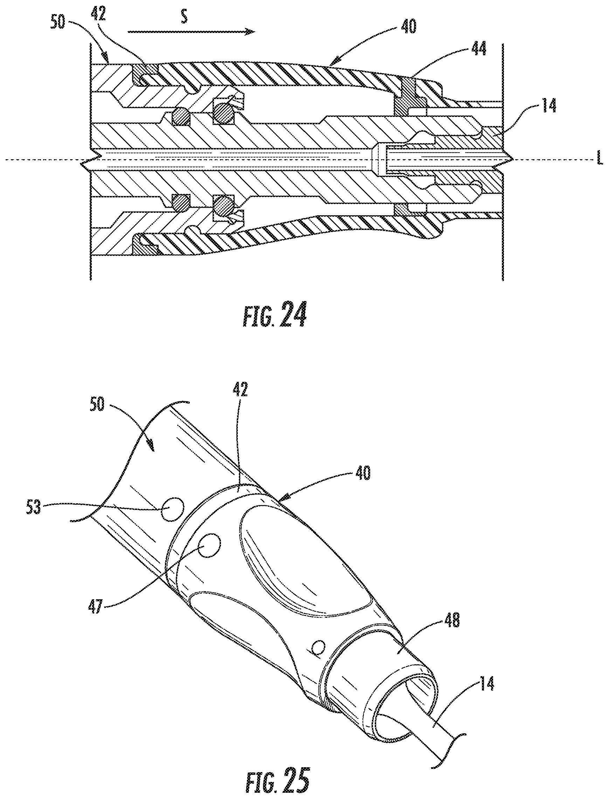

Referring now to FIGS. 24 and 25, attaching the nosecone in some embodiments is simple because the user only needs to install one component without the need for handling separate O-rings. Once an ultrasonic horn 14 has been torqued to a handpiece 12, the nosecone 40 can be slipped over the ultrasonic horn 14. As the nosecone approaches the housing connecting section 52 of the housing 50, the nosecone identification marker 47 on the nosecone 40 should be oriented to align with the connection identification marker 51 on the housing connecting section 52 of the housing 50. This essentially guides the nosecone nubs 43 to the grooves 56 in the housing 50.

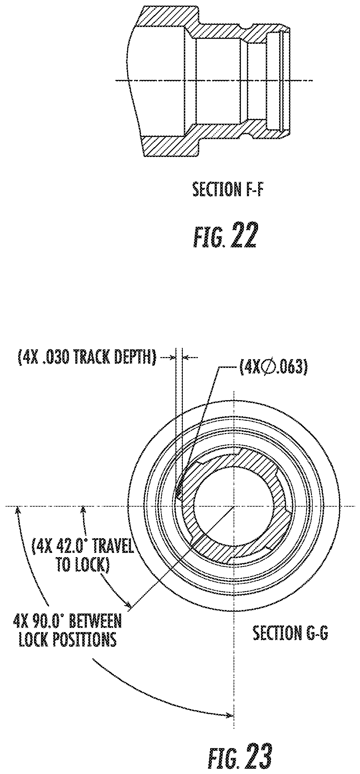

Once the nub 43 has joined the groove 56 of the housing 50, it will follow along and rotate clockwise through the J-shaped track until it reaches the rest position 560, where the end overmold portion 42 compresses. Depending on the orientation of the grooves, the nosecone may travel for a degree to lock. In the exemplary embodiment as shown in FIG. 23, the nosecone travel for about 42 degrees in the grooves to lock.

The nosecone 40 remains fixed in the rest position 560 by means of the expanding of the end overmold portion 42. As shown by arrow S, the end overmold portion 42 acts as a spring, applying a linear force to drive the nub 43 against the end of the groove 56. Locking of nosecone 40 is achieved via elastomer expansion that pushes the nub 43 to contact the wall of the groove 56, for example, the wall in the rest position 560. The flexible property of the thermoplastic material of the end overmold portion 42 works like a spring, expanding out in the longitudinal axis L which results in a linear force to drive the nosecone nubs 43 against the wall at the end of the grooves 56 of the handpiece housing 50 to secure the nosecone firmly to the handpiece.

Although preferably the end overmold portion 42 is an annular piece, it does not have to be a complete annulus. It may have one or more gaps in the ring-shaped structure so long as the end overmold portion provides a spring force that allows locking of the housing 50 and the nosecone 40.

FIG. 25 shows alignment of the housing identification marker 53 on the housing 50 and the nosecone identification marker 47 on the nosecone 40 in fully coupled state. The coupling mechanism is verified to the user via the alignment of the nosecone identification marker 47 to the housing identification marker 53. Once fully assembled, the internal overmold portion 44 conforms to the internal horn 11 and forms a fluid seal to prevent fluid ingress into the handpiece 12.

The internal overmold portion 44 on the inner surface of the nosecone 42 is positioned radially about the nosecone 42 to provide a fluid tight seal that prevents ingress of irrigation fluid into the housing. In addition, the internal overmold portion 44 enables keeping the flue primed, as the surgeon puts the handpiece down on the patient or a table during surgery. The inner seal helps keep the flue primed or irrigation functional immediately, when the handpiece is picked back up by the user. The inner seal reduces the volume that the trickle of irrigation could fill if the system were laid down with the tip up slightly. The inner seal helps keep the liquid in the flue to tip space until use.

An insert molding method can be used for manufacturing the nosecone. In the manufacturing method, a premolded insert is placed into the mold before a thermoplastic material is shot. After the mold assembly is in place, the overmold material is then injected into the mold, either in a semi-fluid or fluid state, using conventional methods known in the art. For making the internal overmold portion, the thermoplastic material can be injected through an opening 45. The manufacturing of the nosecone could be highly economical, such that a mold of the nosecone matrix or substrate is followed by overmolds in a single tool with two or more actuations as needed. The nosecone with overmold portions could be supplied as a disposable, greatly enhancing ease-of-use with simple assembly in the operating room.

Although the exemplary embodiments show the end overmold portion and the internal overmold portion as two separate elements, it is contemplated that the two elements may be two portions of a single piece. The nosecone may have a single overmolded piece that includes an end overmold portion, an internal overmold portion, and a connecting overmold portion that connects the end and internal overmold portions. The connecting overmold portion may be on the inner surface or the outer surface of the nosecone. For example, the nosecone may have an elastomer encased body as a single piece which comprises an end overmold portion, an internal overmold portion, and an elastomer wall on the outer surface of the nosecone. In addition, the elastomer may form part of the nosecone body.

Although one embodiment is illustrated with a J-lock clasp in the handpiece, it is contemplated that the end overmold portions can work with other connecting, coupling or interlocking mechanisms in which O-rings or gaskets are used or desired, such as locking teeth, snap-ears, other snap-in locking features, and other mechanisms. The spring force or seal function necessary to such a connecting, coupling or interlocking mechanism can be achieved through applying an overmolding technology as described in the embodiments of the present invention.

The connection apparatus embodiments of the present invention is useful for attachment of components in machines, apparatuses, instruments and devices including but not limited to medical devices. The nosecone embodiments of the present invention can be applied to various surgical handpieces, such as CUSA Excel 36 kHz and 23 kHz ultrasonic handpieces and other surgical handpieces. The overmolding technology can be applied on longer angle adapted nosecones as well including longer shroud nosecones with angled sections. A person skilled in the art could envision similar requirements and solutions using the basic idea of the embodiments of the present invention. It is contemplated that one, two, or more overmolds may be used depending on the geometry of the handpiece and the needs for sealing and/or connection.

The nosecone and/or flue in some embodiments of the present invention may eliminate user installed O-rings, which are difficult to install for nurses or other users wearing gloves in the sterile field of an operating room. It provides the same critical sealing functionality without cumbersome installation. It eliminates the possibility to install O-rings incorrectly or in the wrong place, and eliminates the problem of assembling the wrong O-rings to surgical tip packs. It may also enable a low cost disposable component created in two actuations of a single molding tool, and enhances ability to provide a clean and sterilized component to the operating room. The nosecone and/or flue in some embodiments of the present invention improves ease of use in assembly, and provides easy and flawless assembly while maintaining functionality. It also enables a clean and sterile component for patient protection in the operating room.

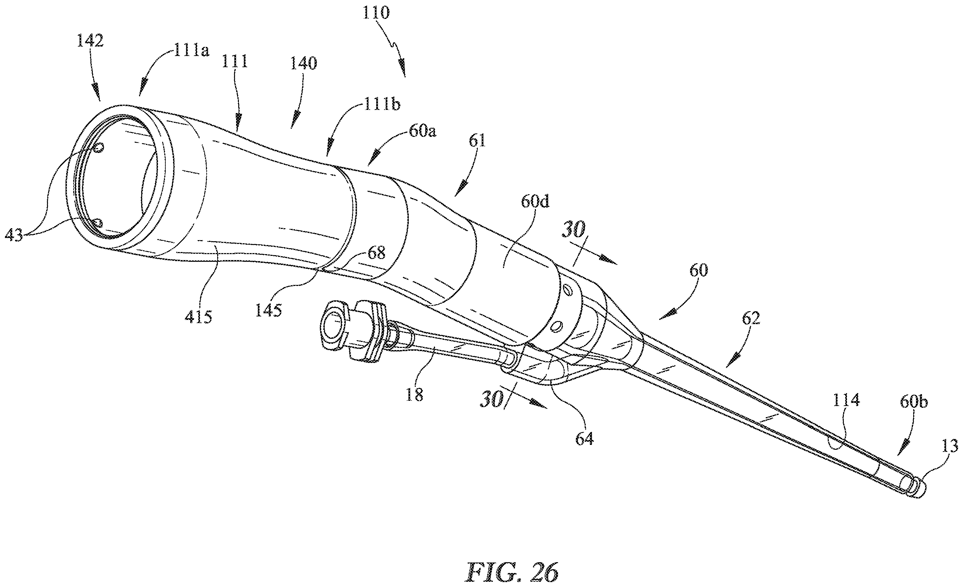

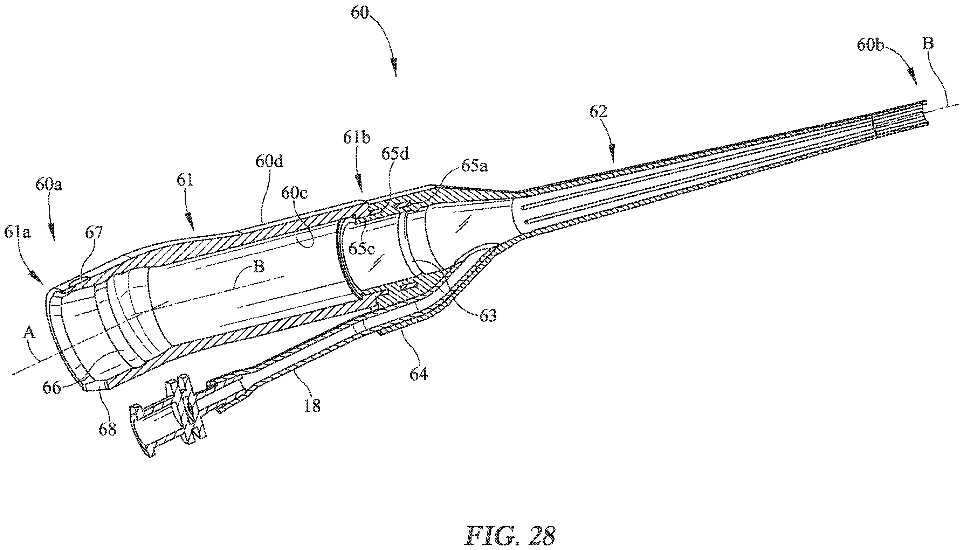

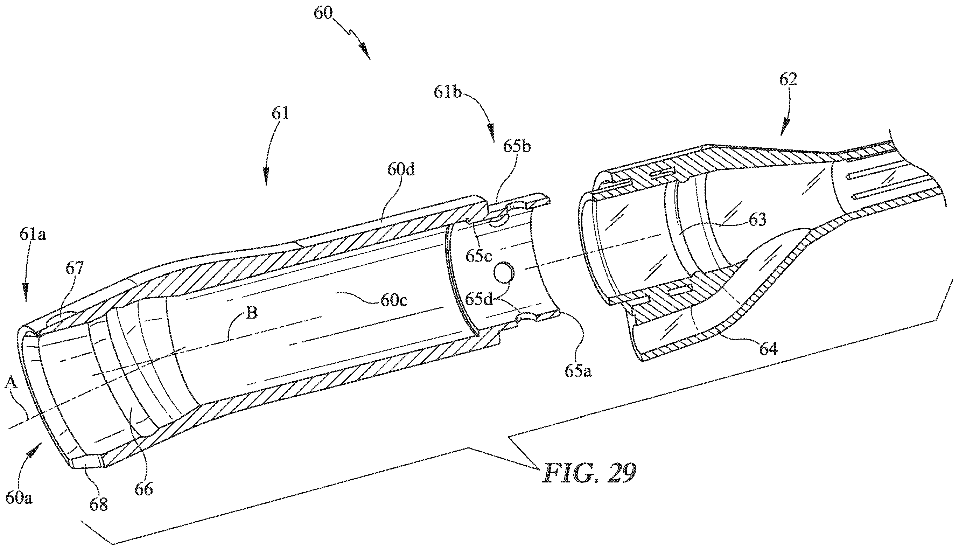

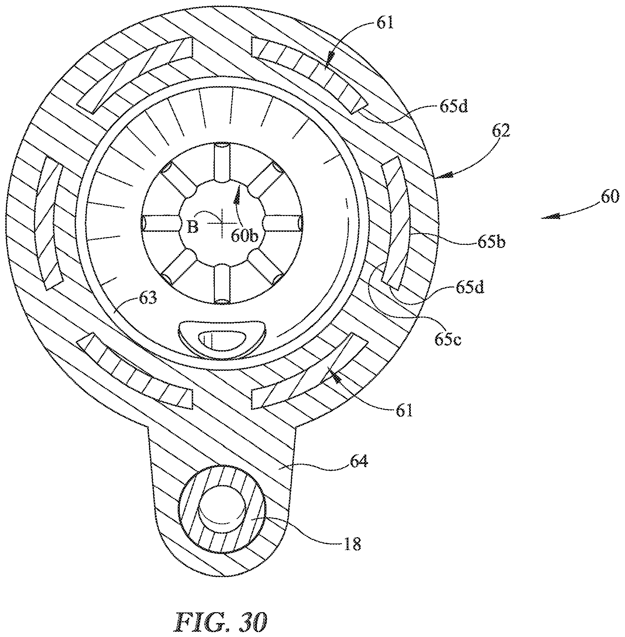

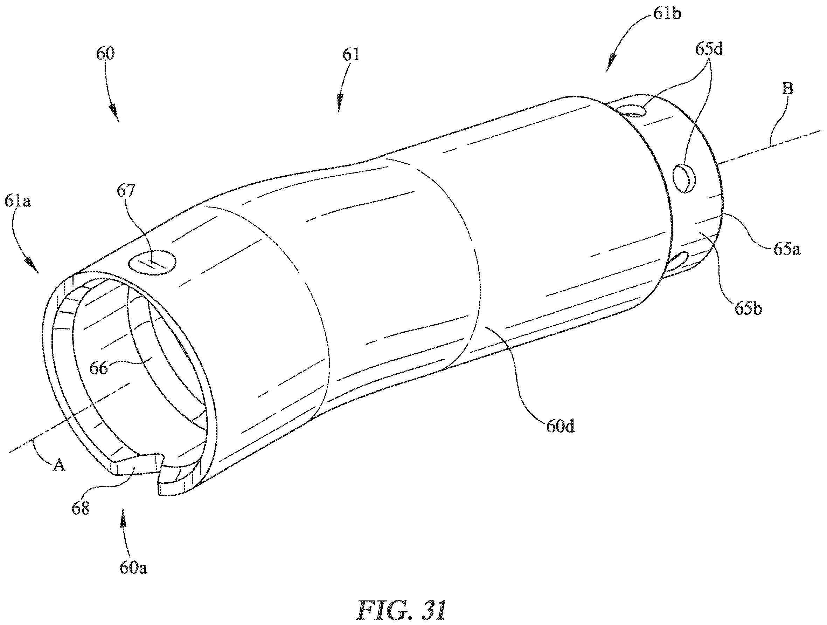

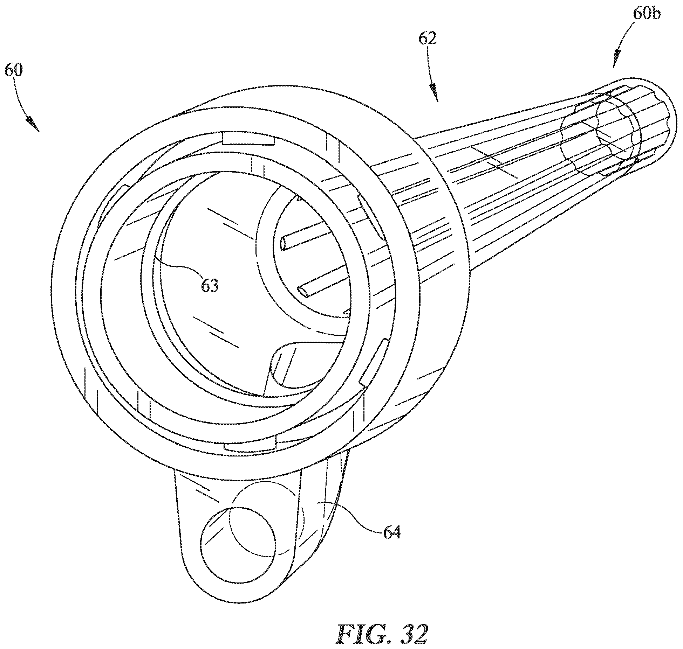

In some implementations, two or more components and/or shots of material may be overmolded together to manufacture a flue and/or a nosecone. The ultrasonic surgical instrument 110 of Fig. 26, illustrates an overmolded flue 60 may extend from the overmolded nosecone 140 towards the end 13 of the surgical tip 114. As shown in FIGS. 26-32, the flue 60 may include at least two shots of material to manufacture the overmolded flue. The body of the flue 60 may include one or more base or base portions 61 and one or more overmold portions 62. The flue 60 may include opposing ends, one end 60a adjacent the nosecone 140 and the other end or free end 60b adjacent the free end 13 of the surgical tip 114 (e.g. bone tip). In the one embodiment shown in FIG. 28, the overmold portion 62 may be adjacent one end 60b of the flue 60 or base 61. It should be understood that the one or more overmold portions 62 may be in a variety of positions along the base 61 or flue 60 (e.g. adjacent the other end 60a of the flue or base, a combination of both ends 60a, 60b, or along the length between the ends). The overmold portion 62 of the flue 60 (e.g. adjacent the base 61) includes an internal protrusion or seal 63 projecting radially inward from an inner surface 60c of the flue 60, base, or overmold portion. The one or more internal protrusions 63 sealingly engage or contact against an outer periphery/circumference of the tip 114 of the instrument 110. The protrusion 63 may have at least one radial protrusion or rib circumscribing the inner circumference of the flue 60 from the inner surface 60c. The overmold portion 62 thereby seals a variety of surgical tips 114 to provide a fluid tight seal that prevents ingress of irrigation fluid into at least a portion of the base 61, headpiece 12, and/or nosecone 140. The overmold portion 62 of the flue 60 may contain a molded irrigation port 64 and/or portion of the flue tube 18, etc. The overmold portion 62 of the flue 60 may narrow from the base 61 in a direction towards the open free end 60b of the flue surrounding or adjacent the surgical tip end 13. In some embodiments, the base 61 may narrow towards the overmold portion 62 of the flue 60 or flue free end 60b.

In some embodiments as shown in FIGS. 28-32, the overmold portion 62 of the flue 60 may engage or contact with a variety of surfaces of the flue base 61. Although the overmold portion 62 of the flue 60 may be positioned or contacts the end face 65a, outer surface 65b, inner surface 65c, and/or through the base body (e.g. apertures 65d), a variety of other engagements or contact surfaces may be used. For example, in some embodiments not shown, the overmold portion 62 or protrusion 63 may extend inwardly from the inner surface 60c or base inner surface 65c and may not go through the aperture 65d, extend over the outer surface, or cover the end face 65a of the base. In other implementations not shown, the overmold portion 62 may not extend through the base wall or aperture 65d and extend only inwardly from or contact the inner surface 65c, 60c. In some embodiments not shown, the overmold portion 62 may extend through the base wall or aperture 65d and project inwardly creating the seal 63 without covering the outer surface 65b or end face 65a. Moreover, the overmold portion may cover the end face 65a and the inner surface 65c in some embodiments. Moreover, although apertures 65d are shown as extending through the base body from the inner surface 65d to the outer surface 65b spaced about the circumference, the apertures may not be used in some embodiments or may not extend completely through the base wall.

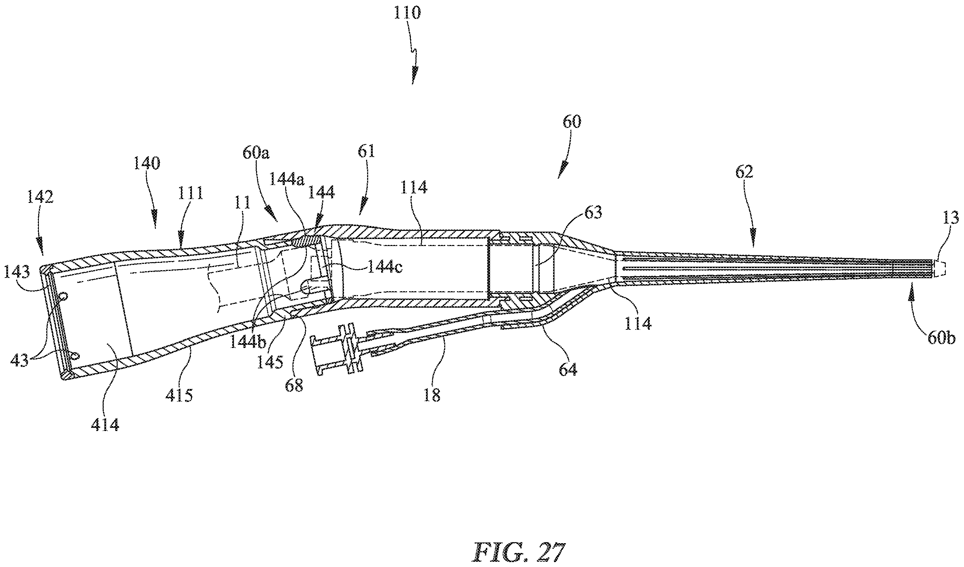

The flue 60 and portions thereof may be a variety of shapes and sizes. In some embodiments as shown more clearly in FIGS. 26 and 27, the flue 60 (e.g. base) may be angled in shape. However, the flue or portions thereof may be substantially straight in various embodiments. The base 61 or portions of the flue 60 may be angled between the opposing ends thereof. The first end 60a of the flue (e.g. base 61 or portions thereof) may extend along a first axis A and the second end 60b of the flue (e.g. the overmold portion 62 or portions thereof) may extend along a second axis B. As shown in FIGS. 27 and 28, the first and second axis A, B are different (e.g. not parallel or transverse to each other). The base 61 includes opposing ends 61a and 61b that may be angled therebetween as shown by axis A and axis B in one embodiment. A variety of angles may be defined by the first and second axis in various applications. In some implementations, the angle and/or narrowing of the base or portions of the flue may allow the user to grip/direct the system to about 90 mm from the surgical site.

The flue 60 and components thereof (e.g. base and/or overmold portions) may be a variety of one or more materials and still be overmolded. The base 61 of the flue 60 may be more rigid than the overmold portion in various embodiments. The flue base 61 may be a rigid polyphenylsulfone (e.g. RADEL brand) or polypropylene in some embodiments. The flue overmold portion 62 may be made from a silicone material in some embodiments. In the embodiment show in FIGS. 26-32, the one or more overmold portions 62 of the flue 60 may be at least partially translucent and/or transparent. For example, the visibility of the overmold portion of the flue 60 (e.g. overmold portion 62) adjacent the working piece or surgical tip 114 may be increased when using a surgical microscope during applications. In some embodiments, the overmold portion 62 of the flue 60 may be more transparent and/or translucent than the base 61.

The one or more overmold portions 62 of the flue 60 may be flexible/elastic in some implementations. Moreover, in some embodiments, the one or more overmold portions 62 of the flue 60 may be more flexible than the base 61 in various embodiments. The flexibility of the flue overmold portions 62 may reduce cracking of the flue overmold portion in some applications (e.g. during applications when working fluid runs out or may be reduced). The flexibility of the overmold portion 62 of the flue 60 or portions thereof may allow for temporarily expanding/expansion of the flue overmold portion to pass over the working tip 114 when connecting the flue 60 to the nosecone 140. For example, when the flue 60 progressively passes over the end 13 of the tip 114 during assembly, the diameter (e.g. tapered end) of the flue overmold portion 62 or portions thereof may stretch or increase in size due to the larger diameter bone tip. In some embodiments, the overmold portion of the flue may subsequently contract or return to or close to its original size/shape, or to a rest position after expanding.

The flue and/or nosecone may connect (e.g. telescopically) in a variety of ways to each other or to a variety of structures of the ultrasonic surgical apparatus 110. In some implementations, the flue 60 and nosecone 140 may engage each other axially and/or rotationally. In the one embodiment, the flue 60 at least axially engages the nosecone 140 between an engaged position (FIGS. 26 and 27) and disengaged positon. The flue base 61 may include one or more grooves 66. The grooves 66 may be positioned within the inner surface 60c or 65c of the flue 60 or base 61. The groove 66 may be a single groove circumscribing the inner surface 65c of the base 61 at the first end 60a of the flue body or base end 61a. The groove 66 may be alternatively, or may be in combination with the inner surface 60c, be positioned on the outer surface 60d of the flue 61d in various embodiment. Although a variety of protrusions or nubs 144 of the nosecone 140 may be shown engaging the groove 66 of the flue base 61, it should be understood that a variety of structure may engage the flue base to releaseably secure the portions of the instrument together. For example, the one or more grooves may be positioned on the nosecone and the flue base may include the one or more protrusions. Moreover, the corresponding structure of the connection between the nosecone and flue may be a combination of the base and/or overmold portions (e.g. first and/or second material shots) of the flue and/or base. For example, an overmold and/or nonovermold portions of the flue may engage an overmold and/or nonovermold portion of the nosecone.

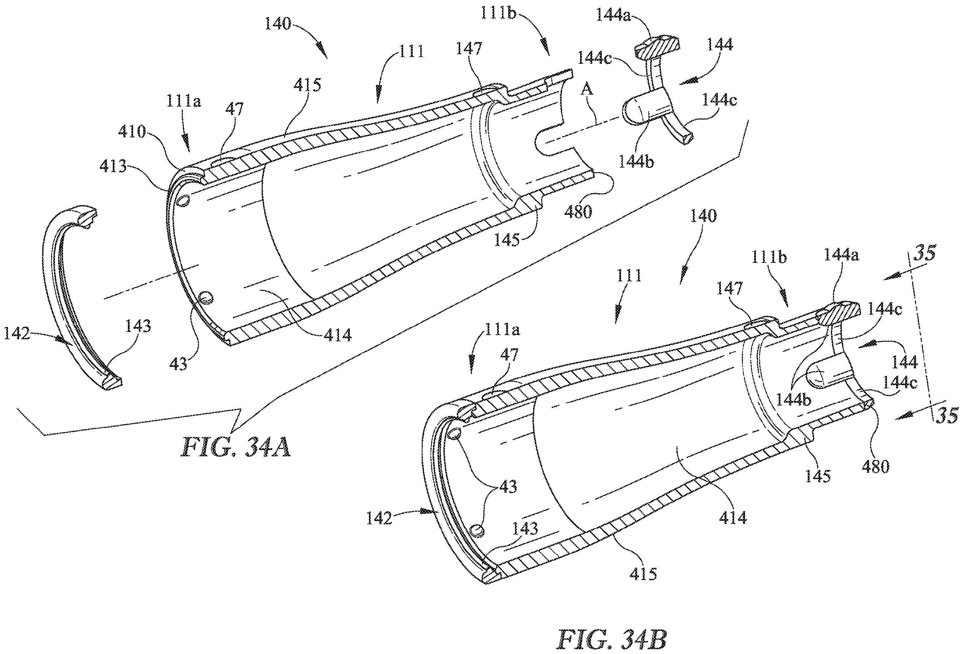

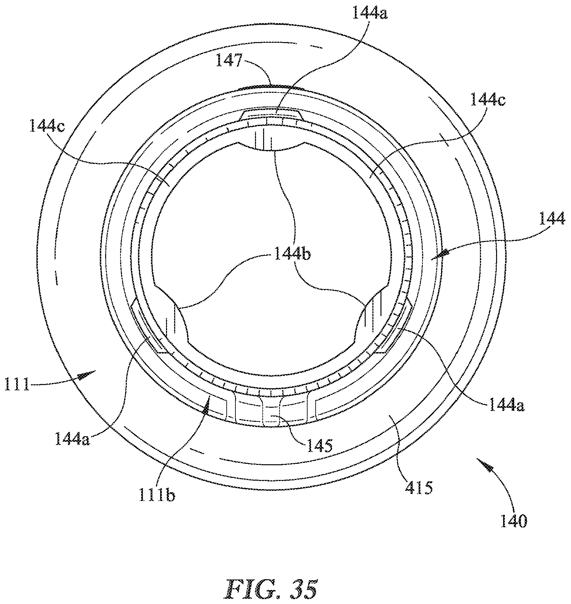

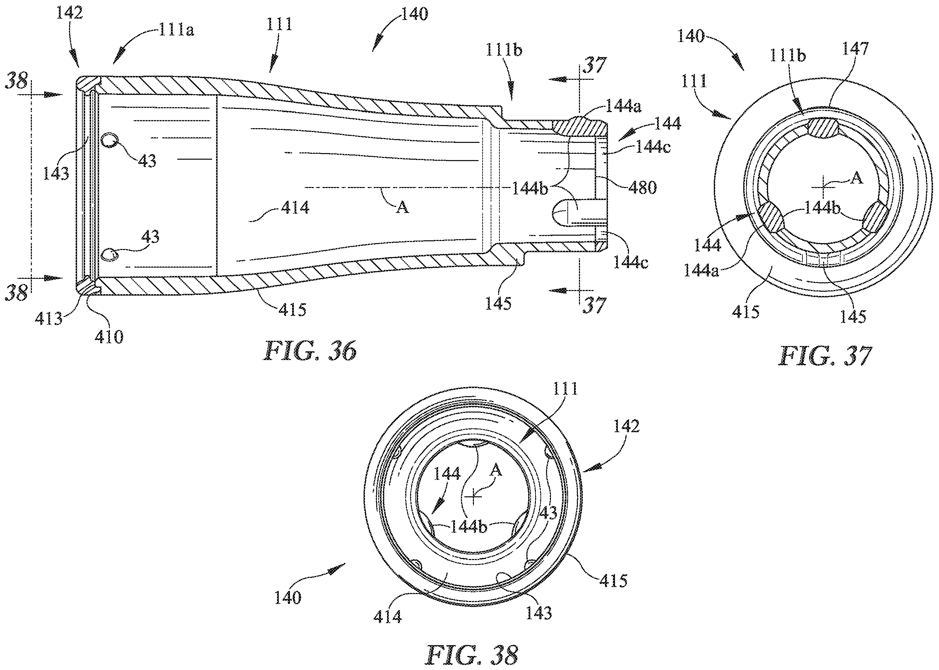

In some implementations as shown in FIGS. 26, 27, and 33-38, the nosecone 140 may include one or more overmold portions releaseably engaging the flue, handpiece, or portions of the apparatus 110. The one or more overmold portions 142, 144, if any, of the nosecone 140 may be overmolded to a base 111 of the nosecone 140. The one or more overmold portions 142, 144 may be positioned on or cover a variety of surfaces (e.g. inner and/or outer surfaces 60c, 60d) of the base 111 and/or along the axis A. The one or more overmold portions 142, 144 of the base 111 may be positioned on opposing ends 111a, 111b of the base 111 (e.g. opposing ends adjacent the handpiece 12 and the flue 60) or nosecone. The one or more overmold portions 142 (e.g. first portions) adjacent the first end 111a of the nosecone or base may engage or contact the handpiece 12. The one or more overmold portions 144 (e.g. second portions) adjacent the second end 111b of the nosecone or base may engage or contact the flue 60 (e.g. flue base) and/or internal horn 11. The overmold portion 142 adjacent the first end 111a may cover an end face 413, lip 410 of the nosecone 140, and/or radially project inwardly from the inner surface 414. The inwardly extending projection 143 of the overmold portion 142 may be a radial seal circumscribing and/or sealing against the handpiece 12 (e.g. the outer surface of the handpiece). The overmold portion 142 covering the end face 413 of the nosecone 140 may be axially compressed adjacent a portion of the handpiece 12. As described above the inner surface 414 of the nosecone may include one or more nubs 43 engaging one or more grooves 56 of the handpiece 12.

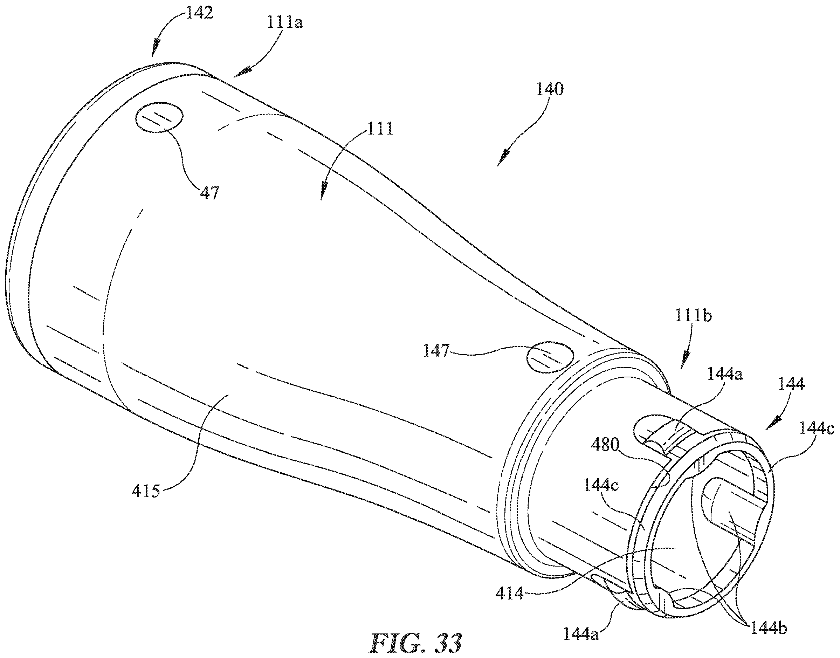

One or more overmold portions of the nosecone 140 may releasably engage the flue 60 (e.g. base) and/or internal horn 11. As shown in FIGS. 33-38, one or more overmold portions 144 extend radially outward from an outer surface 415 of the nosecone 140 or base 111. For example, as shown, one or more first protrusions 144a may extend radially outwardly and engage the groove 66 of the flue 60 or flue base 61. The one or more first protrusions 144a may be spaced about the circumference of the nosecone (e.g. outer surface 415). The one or more portions or protrusions 144b of the overmold portion 144 may extend radially inward from an inner surface 414 of the nosecone 140 or base 111. For example, one or more second protrusions 144b may extend radially inwardly and engage the internal horn 11. The one or more second protrusions 144b may be spaced about the circumference of the nosecone 140 (e.g. inner surface 414). The first and second protrusion 144a, 144b may be interconnected in the embodiment as shown by one or more members 144c. The first and second protrusions 144a, 144b, members 144c, or overmold portions 142, 144 may be a single shot of material although multiple shots may be used to manufacture the nosecone. The one or more overmold portions 142, 144 of the nosecone 140 may cover an end face 413, 480 on the opposing ends 111a, 111b of the base 111 or nosecone 140. The portion or members 144c covering the end face 480 of the second end 111b may interconnect the first and/or second protrusions 144a, 144b. The first and second protrusions 144a and 144b may be spaced similarly about the circumference of the nosecone 140. The first and/or second protrusion 144a, 144b may also be a variety of shapes, sizes, quantities, materials, and positions about the nosecone. For example, the one or more first protrusions 144a may be similar and/or dissimilar in shape, size, quantities, material, and construction to one or more of the second protrusions 144b.

The nosecone and components thereof (e.g. base and/or overmold portions) may be a variety of one or more materials and still be overmolded. In one implementation the base 111 may be a polypropylene and the one or more overmold portions 142, 144 may be a thermoplastic elastomer material (e.g. VERSAFLEX brand soft polymer). In some embodiments, the base 111 of the nosecone 140 may be made from a glass-filled polypropylene. The material of the nosecone and/or flue (e.g. portions thereof) may have compression set, reseal capability, and/or the ability to be sterilized.

There are several identification markers on the apparatus for the coupling mechanisms. The housing 12 has a housing identification marker 53 on the housing body 57 and another connection identification marker 51 on the housing connecting section 52. The nosecone 140 has a nosecone identification marker 47, 147. The flue 60 may include one or more identification markers 67. The identification markers may be in any form, shape and color as desired, such as white dots.

Referring now to FIGS. 26-28, 31, 33, and 34B, attaching the flue and/or horn with the nosecone in some embodiments of the present invention may be without the need for handling separate O-rings. Once the tip 114 has been torqued to the handpiece 12 and/or internal horn 11, the nosecone 140 can be slipped over the ultrasonic horn 114 and connected to the handpiece 12 as described above. As the flue 60 (e.g. end 60a) approaches the connecting section or base end 11b or end of the nosecone 140, the flue identification marker 67 on the flue 60 may be oriented to align with the identification marker 147 on the base 111 of the nosecone 140. This essentially guides the nosecone protrusions 144a to the grooves 66 in the flue 60 as well as engages the protrusions 144b with the internal horn 11. Moreover, in some embodiments, the nosecone and flue may include one or more alignment notch and guide pin engagements therebetween guided by the identification markers 147, 67 to one or more orientations. For example, as shown in FIGS. 26, 27, 31, 35, and 37, the flue 60 includes the notch 68 (e.g. V-shaped slot or receiver) within the end 61a of the base 61 and the nosecone includes the guide pin (e.g. wedge) within the base 111 of the nosecone.

In some implementations, the one or more overmold portions of the nosecone 140 and/or flue 60 may be molded onto and/or through one or more surfaces of a variety of base portions 61, 111. The base portions may be manufactured in a variety of ways and constructions. The base portion 61, 111 of the nosecone 140 and/or flue 60 may be premolded and/or machined before being overmolded by a overmold material. For example, the base portion 61 of the flue 60 may be machined. Alternatively, the base 61 may be molded. In one embodiment, in which the base 61 is machined, the base 61 may be positioned in a cavity and injected with one or more materials (e.g. silicone or soft polymer) to create one or more molded portions 62 thereto. In some embodiments, having a variety of base portions 61, 111 of the nosecone/flue premolded or machined in various quantities,
allows for a variety of designs/constructions of the overmolded portions of the flue/nosecone to be selected and molded to the base portions at a later time for one or more predetermined surgical tip applications. As such, in one embodiment, the base(s) 61 of the flue 60 may allow the same nosecone 140 to be used for a variety of surgical tips merely by selecting between a plurality of flue embodiments having a common base 61 construction and/or connection to the nosecone.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific embodiments described herein. It is, therefore, to be understood that the foregoing embodiments are presented by way of example only and that the invention is defined solely by the claims.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

The embodiments may be embodied in other forms without departure from the scope and essential characteristics thereof. The embodiments described therefore are to be considered in all respects as illustrative and not restrictive. Although the present invention has been described in terms of certain preferred embodiments, other embodiments that are apparent to those of ordinary skill in the art are also within the scope of the invention, which is defined solely by the claims.

## Claims

1. A connection apparatus (110) for attachment of members of a medical device, comprising:
a nosecone (140) having opposing first and second ends (111a, 111b);
a flue (60) having opposing first and second ends (60a, 60b), said flue having a base (61) on said first end (60a);
said second end (111b) of said nosecone (140) releasably engaging said first end (60a) of said flue (60);
**characterised by** said flue (6) having an overmold portion (62) on said second end (60b), and wherein said overmold portion of said flue has a projection (63) extending radially inward from an inner surface (60c) thereof and configured to sealingly engage or contact against an outer periphery or circumference of a surgical tip (114).

2. A connection apparatus as in claim 1, wherein said nosecone (140) includes a first and second overmold portion (142, 144) at each said first end and said second end (111a, 111b), respectively;
and wherein preferably:
said second overmold portion (144) of said nosecone (140) extends both radially outward from an outer surface to engage a groove (66) of said base (61) of said flue (60) and radially inward from an inner surface; and/or,
said second overmold portion (144) of said nosecone (140) at least partially covers an end face (480) of said second end (111b) of said nosecone (140).

3. A connection apparatus as in one of claims 1-2, wherein said overmold portion (62) of said flue includes an irrigation port (64).

4. A connection apparatus as in one of claims 1-3, wherein said overmold portion (62) of said flue is more translucent than said base (61) of said flue.

5. A connection apparatus as in one of claims 1-4, wherein said first end (60a) of said flue extends along a first axis (A) and said second end (60b) of said flue extends along a second axis (B) different from said first axis.

6. An ultrasonic aspirator system for fragmenting tissue and removing fragmented tissue, comprising:
a surgical handpiece (12) comprising a housing, a transducer mounted within the housing, and the connection apparatus (110) of claim 1, wherein the nosecone (140) is attached to the housing, and the flue (60) is attached to the nosecone (140);
a surgical tip (114) connected to the transducer via an internal horn (11);
an irrigation system connected to the handpiece for supplying irrigation fluid adjacent the surgical site for suspending fragmented tissue; and
an aspirating system connected to the handpiece for aspirating fluid and tissue fragmented at the surgical site.

7. An ultrasonic aspirator system as in claim 6, wherein the surgical tip (114) includes a bone tip.

8. An ultrasonic aspirator system as in one of claims 6-7, wherein the overmold portion (62) of the flue tapers towards a free end (60b) of the flue away from the base (61) of the flue.

9. An ultrasonic aspirator system as in one of claims 6-8, wherein the nosecone (140) includes a base (111) and one or more overmold portions (142, 144), and wherein the one or more overmold portions (142, 144) are positioned on opposing ends of the base (111a, 111b);
wherein preferably:
the one or more overmold portions (142, 144) of the nosecone (140) at least one of radially inwardly engages the internal horn (11) and radially outwardly engages the base (61) of the flue (60); and/or,
the one or more overmold portions (142, 144) covers an end face (413, 480) on each one of the opposing ends (111a, 111b) of the base (111) of the nosecone (140).

10. An ultrasonic aspirator system as in one of claims 6-9, wherein the flue (60) includes a groove (66) within the inner surface of the base (61) of the flue (60), wherein the one or more overmold portions (142, 144) of the nosecone (140) engage the groove (66) of the flue (60).

11. A method for attaching members of a medical device, comprising:
providing the connection apparatus (110) of claim 1;
providing a handpiece (12) having a body;
providing a tip (114);
connecting the nosecone (140) to the handpiece (12);
connecting the flue (60) to the nosecone (140); and
sealingly engaging the overmold portion (63) of the flue (60) projecting radially inwardly with the tip (114) to provide a fluid tight seal that prevents ingress of irrigation fluid towards the nosecone (140).

12. A method as in claim 11, wherein when the nosecone (140) is connected to the handpiece (12) and the flue (60), the one or more overmold portions (142, 144) of the nosecone engages:
both the handpiece (12) and the flue (60); and/or,
an internal horn (11).

13. A method as in one of claims 11-12, further comprising the step of temporarily expanding the one or more overmold portions (62) of the flue (60) to pass over the tip (114) when connecting the flue (60) to the nosecone (140).

14. A method as in one of claims 11-13, further comprising the step of contracting the overmold portion (62) to a rest position.

15. A method as in one of claims 11-14, wherein connecting the nosecone (140) to the flue (60) includes engaging the one or more protrusions (144a) of the nosecone with a circumferential groove (66) within the inner surface (60c) of the flue (60).

## Patentansprüche

1. Verbindungseinrichtung (110) zum Anbringen von Elementen einer medizinischen Vorrichtung, umfassend:
einen Nasenkegel (140) mit gegenüberliegenden ersten und zweiten Enden (111a, 111b);
einen Abzugskanal (60) mit gegenüberliegenden ersten und zweiten Enden (60a, 60b), wobei der Abzugskanal am ersten Ende (60a) eine Basis (61) aufweist;
wobei das zweite Ende (111b) des Nasenkegels (140) lösbar mit dem ersten Ende (60a) des Abzugskanals (60) in Eingriff steht;
**dadurch gekennzeichnet, dass** der Abzugskanal (6) einen umspritzten Abschnitt (62) am zweiten Ende (60b) aufweist und wobei der umspritzte Abschnitt des Abzugskanals einen Vorsprung (63) aufweist, der sich von seiner Innenfläche (60c) radial nach innen erstreckt und dazu konfiguriert ist, dichtend mit einem Außenumfang oder Umfang einer chirurgischen Spitze (114) einzugreifen oder diesen zu berühren.

2. Verbindungseinrichtung nach Anspruch 1, wobei der Nasenkegel (140) einen ersten und einen zweiten umspritzten Abschnitt (142, 144) an sowohl dem ersten Ende als auch dem zweiten Ende (111a, 111b) aufweist;
und wobei vorzugsweise:
der zweite umspritzte Abschnitt (144) des Nasenkegels (140) sich sowohl von einer Außenfläche radial nach außen, um in eine Nut (66) der Basis (61) des Abzugskanals (60) einzugreifen, als auch von einer Innenfläche radial nach innen erstreckt; und/oder
der zweite umspritzte Abschnitt (144) des Nasenkegels (140) zumindest teilweise eine Stirnfläche (480) des zweiten Endes (111b) des Nasenkegels (140) bedeckt.

3. Verbindungseinrichtung nach einem der Ansprüche 1-2, wobei der umspritzte Abschnitt (62) des Abzugskanals einen Spülanschluss (64) umfasst.

4. Verbindungseinrichtung nach einem der Ansprüche 1-3, wobei der umspritzte Abschnitt (62) des Abzugskanals lichtdurchlässiger als die Basis (61) des Abzugskanals ist.

5. Verbindungseinrichtung nach einem der Ansprüche 1-4, wobei sich das erste Ende (60a) des Abzugskanals entlang einer ersten Achse (A) erstreckt und sich das zweite Ende (60b) des Abzugskanals entlang zweiten Achse (B) erstreckt, die sich von der ersten Achse unterscheidet.

6. Ultraschallabsaugsystem zum Fragmentieren von Gewebe und Entfernen von fragmentiertem Gewebe, umfassend:
ein chirurgisches Handstück (12), das ein Gehäuse, einen im Gehäuse montierten Wandler und die Verbindungseinrichtung (110) nach Anspruch 1 umfasst, wobei der Nasenkegel (140) am Gehäuse befestigt ist und der Abzugskanal (60) am Nasenkegel (140) befestigt ist;
eine chirurgische Spitze (114), die über ein internes Horn (11) mit dem Wandler verbunden ist;
ein Spülsystem, das mit dem Handstück verbunden ist, um Spülfluid neben der Operationsstelle zuzuführen und fragmentiertes Gewebe zu suspendieren; und
ein mit dem Handstück verbundenes Absaugsystem zum Absaugen von Fluid und fragmentiertem Gewebe an der Operationsstelle.

7. Ultraschallabsaugsystem nach Anspruch 6, wobei die chirurgische Spitze (114) eine Knochenspitze umfasst.

8. Ultraschallabsaugsystem nach einem der Ansprüche 6-7, wobei sich der umspritzte Abschnitt (62) des Abzugskanals zu einem freien Ende (60b) des Abzugskanals hin verjüngt, das von der Basis (61) des Abzugskanals entfernt ist.

9. Ultraschallabsaugsystem nach einem der Ansprüche 6-8, wobei der Nasenkegel (140) eine Basis (111) und einen oder mehrere umspritzte Abschnitte (142, 144) beinhaltet und wobei der eine oder die mehreren umspritzten Abschnitte (142, 144) an gegenüberliegenden Enden der Basis (111a, 111b) positioniert sind;
wobei vorzugsweise:
der eine oder die mehreren umspritzten Abschnitte (142, 144) des Nasenkegels (140) mindestens entweder radial nach innen in das interne Horn (11) eingreifen oder radial nach außen in die Basis (61) des Abzugskanals (60) eingreifen; und/oder,
der eine oder die mehreren umspritzten Abschnitte (142, 144) eine Stirnfläche (413, 480) an jedem der gegenüberliegenden Enden (111a, 111b) der Basis (111) des Nasenkegels (140) abdecken.

10. Ultraschallabsaugsystem nach einem der Ansprüche 6-9, wobei der Abzugskanal (60) eine Nut (66) in der Innenfläche der Basis (61) des Abzugskanals (60) aufweist, wobei der eine oder die mehreren umspritzten Abschnitte (142, 144) des Nasenkegels (140) in die Nut (66) des Abzugskanals (60) eingreifen.

11. Verfahren Anbringen von Elementen einer medizinischen Vorrichtung, umfassend:
Bereitstellen der Verbindungseinrichtung (110) nach Anspruch 1;
Bereitstellen eines Handstücks (12) mit einem Körper;
Bereitstellen einer Spitze (114);
Verbinden des Nasenkegels (140) mit dem Handstück (12);
Verbinden des Abzugskanals (60) mit dem Nasenkegel (140); und
abdichtendes Eingreifen des umspritzten Abschnitts des Abzugskanals (60), der radial nach innen vorsteht, mit der Spitze (114), um eine fluiddichte Abdichtung bereitzustellen, die Eindringen von Spülfluid in Richtung des Nasenkegels (140) verhindert.

12. Verfahren nach Anspruch 11, wobei, wenn der Nasenkegel (140) mit dem Handstück (12) und dem Abzugskanal (60) verbunden ist, der eine oder die mehreren umspritzten Abschnitte (142, 144) des Nasenkegels mit Folgendem eingreifen:
sowohl dem Handstück (12) als auch dem Abzugskanal (60); und/oder
einem internen Horn (11).

13. Verfahren nach einem der Ansprüche 1-12, weiter umfassend den Schritt zum vorübergehenden Erweitern des einen oder der mehreren umspritzten Abschnitte (62) des Abzugskanals (60), um über die Spitze (114) geführt werden, wenn der Abzugskanal (60) mit dem Nasenkegel (140) verbunden wird.

14. Verfahren nach einem der Ansprüche 11-13, das weiter den Schritt zum Zusammenziehen des umspritzten Abschnitts (62) in eine Ruheposition umfasst.

15. Verfahren nach einem der Ansprüche 11-14, wobei Verbinden des Nasenkegels (140) mit dem Abzugskanal (60) das Eingreifen des einen oder der mehreren Fortsätze (144a) des Nasenkegels mit einer Umfangsnut (66) innerhalb der Innenfläche (60c) des Abzugskanals (60) umfasst.

## Revendications

1. Appareil de connexion (110) pour la fixation d'éléments d'un dispositif médical, comprenant :
une coiffe (140) présentant des première et seconde extrémités (111a, 111b) opposées ;
un conduit (60) présentant des première et seconde extrémités (60a, 60b) opposées, ledit conduit présentant une base (61) sur ladite première extrémité (60a) ;
ladite seconde extrémité (111b) de ladite coiffe (140) mettant en prise amovible ladite première extrémité (60a) dudit conduit (60) ;
**caractérisé par** ledit conduit (6) présentant une partie surmoulée (62) sur ladite seconde extrémité (60b), et dans lequel ladite partie surmoulée dudit conduit présente une saillie (63) s'étendant radialement vers l'intérieur à partir d'une surface intérieure (60c) de celui-ci et configurée pour venir en prise ou en contact de manière étanche avec une périphérie ou circonférence extérieure d'une pointe chirurgicale (114).

2. Appareil de connexion selon la revendication 1, dans lequel ladite coiffe (140) inclut une première et une seconde partie surmoulée (142, 144) au niveau de chacune desdites première extrémité et seconde extrémité (111a, 111b), respectivement ;
et dans lequel de préférence :
ladite seconde partie surmoulée (144) de ladite coiffe (140) s'étend à la fois radialement vers l'extérieur à partir d'une surface extérieure pour venir en prise dans une rainure (66) de ladite base (61) dudit conduit (60) et radialement vers l'intérieur à partir d'une surface intérieure ; et/ou,
ladite seconde partie surmoulée (144) de ladite coiffe (140) recouvre au moins partiellement une face d'extrémité (480) de ladite seconde extrémité (111b) de ladite coiffe (140).

3. Appareil de connexion selon l'une des revendications 1-2, dans lequel ladite partie surmoulée (62) dudit conduit inclut un orifice d'irrigation (64).

4. Appareil de connexion selon l'une des revendications 1-3, dans lequel ladite partie surmoulée (62) dudit conduit est plus translucide que ladite base (61) dudit conduit.

5. Appareil de connexion selon l'une des revendications 1-4, dans lequel ladite première extrémité (60a) dudit conduit s'étend le long d'un premier axe (A) et ladite seconde extrémité (60b) dudit conduit s'étend le long d'un second axe (B) différent dudit premier axe.

6. Système d'aspiration à ultrasons pour fragmenter un tissu et retirer un tissu fragmenté, comprenant :
une pièce à main chirurgicale (12) comprenant un boîtier, un transducteur monté à l'intérieur du boîtier, et l'appareil de connexion (110) selon la revendication 1, dans lequel la coiffe (140) est fixée au boîtier, et le conduit (60) est fixé à la coiffe (140) ;
une pointe chirurgicale (114) connectée au transducteur par le biais d'une corne interne (11) ;
un système d'irrigation connecté à la pièce à main pour fournir un fluide d'irrigation de manière adjacente au site chirurgical pour suspendre le tissu fragmenté ; et
un système d'aspiration connecté à la pièce à main pour aspirer le fluide et le tissu fragmenté au niveau du site chirurgical.

7. Système d'aspiration à ultrasons selon la revendication 6, dans lequel la pointe chirurgicale (114) inclut une pointe osseuse.

8. Système d'aspiration à ultrasons selon l'une des revendications 6-7, dans lequel la partie surmoulée (62) du conduit s'effile vers une extrémité libre (60b) du conduit loin de la base (61) du conduit.

9. Système d'aspiration à ultrasons selon l'une des revendications 6-8, dans lequel la coiffe (140) inclut une base (111) et une ou plusieurs parties surmoulées (142, 144), et dans lequel les une ou plusieurs parties surmoulées (142, 144) sont positionnées sur des extrémités opposées de la base (111a, 111b) ;
dans lequel de préférence :
les une ou plusieurs parties surmoulées (142, 144) de la coiffe (140) effectuent au moins l'une des actions parmi venir en prise radialement vers l'intérieur avec la corne interne (11) et venir en prise radialement vers l'extérieur avec la base (61) du conduit (60) ; et/ou,
les une ou plusieurs parties surmoulées (142, 144) recouvrent une face d'extrémité (413, 480) sur chacune des extrémités (111a, 111b) opposées de la base (111) de la coiffe (140).

10. Système d'aspiration à ultrasons selon l'une des revendications 6-9, dans lequel le conduit (60) inclut une rainure (66) à l'intérieur de la surface intérieure de la base (61) du conduit (60), dans lequel les une ou plusieurs parties surmoulées (142, 144) de la coiffe (140) viennent en prise avec la rainure (66) du conduit (60).

11. Procédé de fixation d'éléments d'un dispositif médical, comprenant les étapes consistant à :
fournir l'appareil de connexion (110) selon la revendication 1 ;
fournir une pièce à main (12) présentant un corps ;
fournir une pointe (114) ;
connecter la coiffe (140) à la pièce à main (12) ;
connecter le conduit (60) à la coiffe (140) ; et
mettre en prise de manière étanche la partie surmoulée du conduit (60) faisant saillie radialement vers l'intérieur avec la pointe (114) pour fournir un joint étanche aux fluides qui empêche l'entrée de fluide d'irrigation vers la coiffe (140).

12. Procédé selon la revendication 11, dans lequel lorsque la coiffe (140) est connectée à la pièce à main (12) et au conduit (60), les une ou plusieurs parties surmoulées (142, 144) de la coiffe viennent en prise avec :
à la fois la pièce à main (12) et le conduit (60) ; et/ou,
une corne interne (11).

13. Procédé selon l'une des revendications 11-12, comprenant en outre l'étape consistant à dilater temporairement les une ou plusieurs parties surmoulées (62) du conduit (60) pour passer par-dessus la pointe (114) lors de la connexion du conduit (60) à la coiffe (140).

14. Procédé selon l'une des revendications 11-13, comprenant en outre l'étape consistant à contracter la partie surmoulée (62) vers une position de repos.

15. Procédé selon l'une des revendications 11-14, dans lequel l'étape consistant à connecter la coiffe (140) au conduit (60) inclut l'étape consistant à mettre en prise les une ou plusieurs saillies (144a) de la coiffe avec une rainure circonférentielle (66) à l'intérieur de la surface intérieure (60c) du conduit (60).
